# EUROPEAN PATENT APPLICATION

(11) **EP 1 136 095 A1**
(43) Date of publication of application: **26.09.2001**
(21) Application number: 01201041.9
(22) Date of filing: 20.03.2001
(51) Int. Cl.: A61M 25/06

(54) **Casing for housing an injection needle device after use**

(30) Priority: 21.03.2000 IT MI000585
(71) Applicant: ZAMBON GROUP S.p.A., 36100 Vicenza (IT)
(72) Inventor: Zambon, Andrea Alessandro, 20121 Milano (IT); Zannini, Gaetano, 36023 Longare (VI) (IT); Guiges, Joel, 06300 Nizza (FR)
(74) Representative: Marchi, Massimo

(57) **Abstract**

A casing (1) for housing a device (25) with an injection needle (21) after use, comprises:
a) a first lobe (1a) and a second lobe (1b) connected together by means of a weakening line (2), said line allowing folding of one of said first and second lobes (1a,1b) onto the other one,
b) at least one depression (3a,3b) on the bottom side of said first and second lobes (1a,1b), said depression (3a,3b) being situated in the vicinity of said weakening line (2) and being able to receive firmly said device (25) when said first and second lobes (1a,1b) are folded one onto the other.

## Description

The present invention relates to a casing for housing an injection needle device after use.

More particularly, the present invention relates to a casing for an injection needle device of the known type comprising a sheath which slidably houses an injection needle connected to a small pipe supplying the solution to be injected, said needle also being connected integrally to two flexible projections - also called "butterfly wings" - which project from said sheath through two longitudinal side openings inside which they slide so as to displace said needle from the inside towards the outside of said sheath and vice versa.

At present a very pressing requirement is to prevent a health worker from coming into direct contact with a patient's blood, in order to avoid any possible risk of infection.

At present, a simple and effective means for preventing the worker from coming into contact with the patient's blood, during use of an injection device of the known type described above, is not known to exist.

The present invention aims to overcome the abovementioned drawback by means of a casing for housing an injection needle device after use, characterized in that it comprises:
a) a first lobe and a second lobe connected together by means of a weakening line, said line allowing folding of one of said first and second lobes onto the other one,
b) at least one depression on the bottom side of said first and second lobes, said depression being situated in the vicinity of said weakening line and being able to receive firmly said device when said first and second lobes are folded one onto the other,
c) a first and a second wing in the front part of said first and second lobe,
d) a first and a second lug in the rear part of said first and second lobe,
e) at least one protruding projection on the bottom side of one of said first and second wings and first and second lugs,
f) at least one recess on the bottom side of one of said first and second wings and first and second lugs, said at least one recess being able to co-operate with said at least one protruding projection so as to retain said first and second lobes in the position folded one onto the other.

Preferably, said casing is characterized in that said first and second wings have, on the top side, a hollow able to facilitate gripping of said wings by the worker.

The casing of the present invention solves the problem of preventing the worker from coming into contact with the patient's blood and has the advantage that it is easy to manufacture and easy to use.

Further characteristic features and advantages of the invention will now be illustrated with reference to an embodiment provided only by way of example with reference to the enclosed figures.

Figure 1 is a view, from above, of the top side of a casing according to the present invention in the open position.

Figure 2 is a front view of the casing of Figure 1.

Figure 3 is a cross-sectional view along the plane indicated by III-III in Figure 1.

Figure 4 is a cross-sectional view along the plane indicated by IV-IV in Figure 1.

Figure 5 is a front view of the casing of Figure 1 in the closed position.

Figure 6 is a front view of the casing of Figure 1 in the semi-open position.

Figure 7 is a view, from above, of a device of the known type provided with a butterfly injection needle and cylindrical sheath able to house slidably said needle.

Figure 8 is a view, from above, of the device according to Figure 7 when the butterfly needle is fully housed inside said cylindrical sheath.

Figure 9 is a perspective view of the bottom side of the casing of Figure 1 in the open position, associated with the device of Figure 8.

Figure 10 illustrates the operation required in order to close the device of Figure 8 inside the casing of Figure 1.

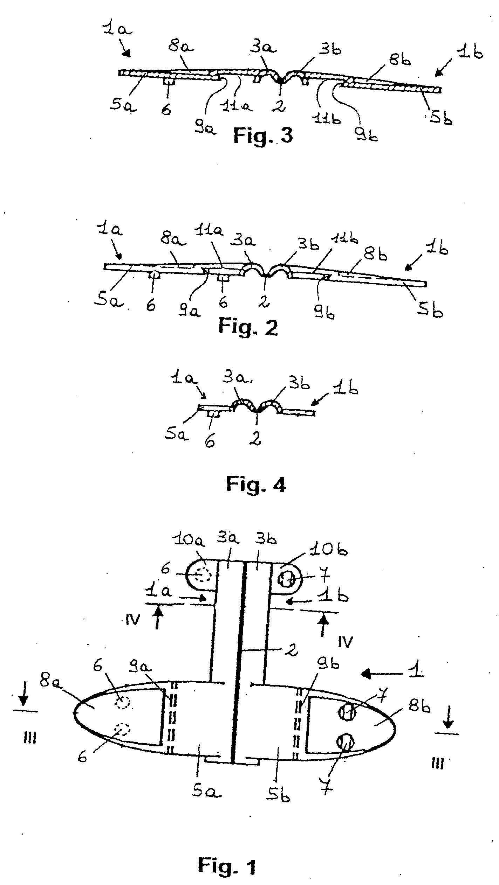

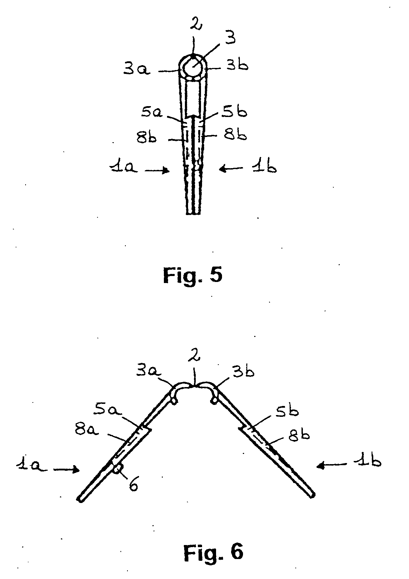

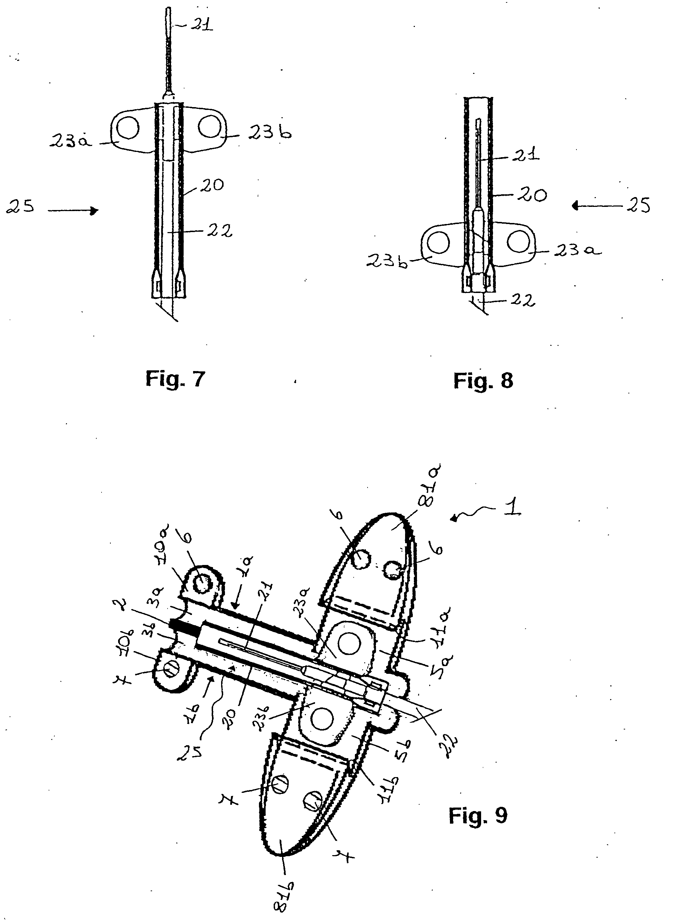

As illustrated in Figures 1 - 6 and 9, an embodiment of a casing (1) according to the present invention for housing an injection needle device after use comprises a first lobe (1a) and a second lobe (1b) which are connected together by means of a weakening line or groove (2) which allows folding of one of said first and second lobes (1a, 1b) onto the other. Each of said first and second lobes (1a, 1b) has, in the vicinity of said groove (2), a depression (3a, 3b). When said casing is closed [Figure 5], said depressions (3a, 3b) form a substantially cylindrical longitudinal cavity (3) which is able to contain and retain firmly an injection device such as, for example, that shown in Figures 7 and 8.

The front part of each of said first and second lobes (1a, 1b) is in the form of a wing (5a, 5b). Each wing (5a, 5b) has a hollow (8a, 8b); said hollows (8a, 8b) being able to receive the thumb and the index finger, respectively, of the hand used by the worker.

The rear part of each of said first and second lobes (1a, 1b) is in the form of a lug (10a, 10b).

The bottom sides of a wing (5a) and a lug (10a) are provided with protruding projections (6) able to co-operate with corresponding recesses (7) formed on the bottom sides of the other wing (5b) and the other lug (10b), respectively, so as to keep the casing (1) according to the present invention in the closed position [Figure 5]. Moreover, the bottom side of said wings (5a, 5b) is provided with inclined walls (9a, 9b) which form a seat (11a, 11b).

Figures 7 and 8 show a device (25) of the known type comprising a sheath (20) which slidably houses an injection needle (21) connected to a small pipe (22) supplying the solution to be injected. Said needle (21) is also connected integrally to two flexible projections (23a, 23b) - also called "butterfly wings" - which project from the sheath (20) through two longitudinal side openings (not shown) inside which they slide so as to displace said needle (21) from the inside to the outside of said sheath (20) and vice versa.

As shown in Figure 9, the bottom side of each wing (5a, 5b) has raised portions (81a, 81b), the side of which directed towards the depressions (3a, 3b) is inclined so as to create the seat (11a, 11b) able to engage with the outer ends of the flexible projections (23a, 23b) of the device (25).

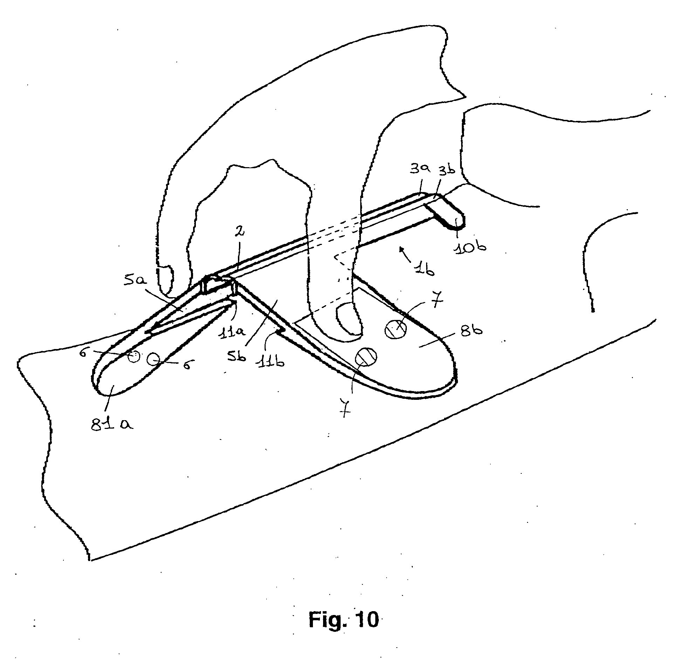

Figure 10 shows how the casing (1) is gripped by the worker.

During use, after performing an injection and while the needle (21) is still inserted in the patient's body, the worker must place the casing (1), in the open position, over the device (25) so that said projections (23a, 23b) are located substantially aligned with wings (5a, 5b) as shown in Figure 9.

Then, the worker slowly withdraws said casing exerting a light pressure on the wings (5a, 5b) in order to keep the projections (23a, 23b) engaged in the seats (11a, 11b). In this way, the needle (21) is extracted from the patient's body and caused to slide inside the sheath (20) of the device (25). When the needle (21) is fully housed inside the sheath (20), the worker folds one lobe (1a, 1b) onto the other one. Finally, the worker exerts a suitable pressure on the wings (5a, 5b) and the lugs (10a, 10b) in order to cause the protruding projections (6) to enter into the corresponding recesses (7). The casing thus remains closed with the device (25) firmly housed inside the cylindrical cavity (3) and may be disposed of without the worker running any risk of coming into contact with the blood-contaminated needle.

## Claims

1. A casing (1) for housing a device (25) with an injection needle (21) after use, **characterized in that** it comprises:
a) a first lobe (1a) and a second lobe (1b) connected together by means of a weakening line (2), said line allowing folding of one of said first and second lobes (1a, 1b) onto the other one,
b) at least one depression (3a, 3b) on the bottom side of said first and second lobes (1a, 1b), said depression (3a, 3b) being situated in the vicinity of said weakening line (2) and being able to receive firmly said device (25) when said first and second lobes (1a, 1b) are folded one onto the other,
c) a first and a second wing (5a, 5b) in the front part of said first and second lobes (1a, 1b),
d) a first and a second lug (10a, 10b) in the rear part of said first and second lobes (1a, 1b),
e) at least one protruding projection (6) on the bottom side of one of said first and second wings (5a) and first and second lugs (10a),
f) at least one recess (7) on the bottom side of one of said first and second wings (5a) and first and second lugs (10a), said at least one recess (7) being able to co-operate with said at least one protruding projection (6) so as to retain said first and second lobes (1a, 1b) in the position folded one onto the other.

2. A casing (1) according to Claim 1, **characterized in that** said first and second wings (5a, 5b) have, on the top side, a hollow (8a, 8b) able to facilitate gripping of said wings by the worker.
